# EUROPEAN PATENT APPLICATION

(11) **EP 0 683 984 A1**
(43) Date of publication of application: **29.11.1995**
(21) Application number: 93922103.2
(22) Date of filing: 16.09.1993
(51) Int. Cl.: A23C 9/12

(54) **BIOLOGICALLY ACTIVE SOURED MILK PRODUCT "ACIDOLACT-NARINEH" AND A PROCESS FOR PRODUCING THE SAME**

(30) Priority: 05.02.1993 RU 9302600
(71) Applicant: Parsekian, Harry, Watertown, Massachusetts 02172 (US)
(72) Inventor: IZVEKOVA, Tamara Georgievna, Smolenskaia obl., 215810 (RU); KORNILOV, Alexandr Viktorovitch, Smolenskaia obl., 215810 (RU); AMIRIAN, Irina Surenovna, Erevan, 375044 (AM)
(74) Representative: W.P. Thompson & Co.
(86) International application number: PCT/RU93/00220
(87) International publication number: WO 94/17671

(57) **Abstract**

A biologically active soured milk product "acidolact-narineh" is disclosed containing 0.35-34.0 wt.% protein, 0.05-36.0 wt.% fats, 1.5-70 wt.% carbohydrates, 0.6-56 wt.% organic acids (lactic acid equivalent), 0.95-15.90 mg of vitamins per 100 g or product, 4.0-96.6 wt.% water, and between 10⁶ and 10⁹ symbiotic microorganisms of the species Lactobacterium acidophilum per gram of the product, there being at least one strain of the group Ep-2 with the strain N.V. Ep 317/402, the ratio between the said strains being 1:1-4 as appropriate. The acidity of the product is 60 to 280 ^{o}T. The energy value of the product is 20 to 540 kilocalories per 100 g. This biologically active soured milk product is obtained by heat-treating the milk at a temperature of 95 to 140 ^{o}C and then cooling it to fermentation temperature, introducing microorganisms of the species Lactobacterium acidophilum with at least one strain of group Ep-2 and the strain N.V. Ep 317/402, the quantity of the Ep-2 strain being such that the ratio between it and the strain N.V. Ep 317/402 is 1:1 to 4 as appropriate. Once the microorganisms have been introduced, fermentation is allowed to continue until acidity reaches 60 to 280 ^{o}T.

## Description

### Technical Field

The present invention relates in general to dairy industry, and is particularly concerned with a biologically active fermented-milk product "Acidolact-Narineh" and a process for its production. It can also find application in medical and veterinary practice as a treatment-and-prevention preparation.

### Background of the Invention

Milk of various animals is processed into fermented milk products in all countries throughout the world, which differ not only in the milk origin (cow, sheep, mare, goat, etc.) but also in a process for its production, organoleptic properties, a chemical composition and spontaneous microflora.

Symbiosis of lactic acid bacteria (such as Lactobacterium bulgaricum, Streptococcus thermophilus, Thermobacterium bulgaricum) and, probably, of yeast-like fungi is a principal inducer of lactic acid fermentation. A pleasant taste and easy digestibility are common and characteristic for all kinds of a fermented milk. Fermented-milk products favour a better digestion and assimilation of the food components. These products possess a high caloric value, dietary and curative properties in treatment of various diseases of a non-infectious and infectious nature.

At the same time, the acidophilic-type lactic-acid bacteria are known, against other lactic acid bacteria, to be better adaptable for a more prolonged period in the large intestine of humans, while a higher biological activity of Lactobacillus acidophilus is primarily associated with its ability to produce a substantial amount of antimicrobial substances harmless to humans and particularly to infants.

A fermented milk prepared with Lactobacillus acidophilus possesses high nutritive and curative properties and is also a stimulant of not only gastric secretion but generally of a motor-secretory function of the whole gastrointestinal tract.

The composition of a soured product includes microorganisms of Lactobacterium acidophilum species in an amount of 10⁷ - 10⁸ bacteria per gram of the product, 2.8 wt.% protein, 3.5 wt.% fats, 88.5 wt.% water, 1173 mg of essential amino acids per liter (with a scaling factor of 6.38), namely valine (157), isoleucine (156), leucine (267), lysine (215), methionine (71), threonine (126), tryptophan (41), phenylalanine (140), and nonessential aminoacids, namely, alanine (80), arginine (100), aspartic acid (179), histidine (74), glycine (38), glutamic acid (492), proline (248), serine (153), tyrosine (151), cystine (20).

Some acidophilic bacteria are known as flavin producers and are integrated into a group called Ep-2 pertaining to Lactobacterium acidophilum species, for example, strain No. 317/381, 317/393, 317/401 or 317/389 (of L.A.Erzinkyan; Biological Features of Some Lactic Acid Bacteria Strains, Publishing House of the Academy of Sciences of the Armenian Republic, Yerevan, 1971, pp. 79-96, 172).

Bacteria of Lactobacterium acidophilum species making part of the abovesaid group are microaerophilic, Gram-positive, homofermentative, immobile, appearing as granular and agranular asporogenic bacteria with the size of 2 - 10 * 0.7 - 0.8 µm.

Cells are disposed singly or in short or long chains, they do not reduce nitrates and do not form indole and skatole nor dilute gelatine. The bacteria display no hemolytic activity. They are able to ferment the following substances: lactose, glucose, sucrose, fructose, mannose, levulose, galactose, maltose, raffinose, starch, dextrin, sorbitol, mannitol, dulcitol. They do not ferment inositol. When grown on selective agar nutrient media in Burri tubes and Petri dishes, they form subsurface colonies which are shallow, rarely smooth (S-forms), most commonly in the form of a ball of entangled threads (R-forms). A minimum growth temperture is above 20 ^{o}C, an optimum temperature is 35 - 39 ^{o}C, a lethal temperature is 68 - 70 ^{o}C. A maximum acidification in milk is 300 - 360 ^{o}T, a phenol resistance in milk is 0.9 - 1.0 %, a phthalazo-resistance - 0.9 - 1.0 %, a synthomycin resistance - 0.003 %.

Microorganisms of Lactobacterium acidophilum species, of the strains of group Ep-2, are capable of producing a substantial amount of antimicrobial substances which are harmless to humans and especially to a child's organism, wherein these substances suppress the growth and development of Gram-positive (Staphilococcus aureus) and Gram-negative (Escherichia coli 113) bacteria including all, without an exception, bacteria-pathogens causative of dysentery and dysentery-like diseases. Depending on a souring temperature and the amount of the ferment introduced, a period of milk clotting varies from 4 to 8 hours.

Based on the aforementioned acidophilic milk souring, special food products have been developed for a compound and artificial feeding of healthy infants, as well as premature infants with the phenomena of hypotrophy, with gastrointestinal tract's diseases.

To manufacture such products, the K1, K5, K10 strains of Lactobacillum acidophilum, cow milk, seed oil, refined sugar are used. The products thus obtained are enriched with vitamins A, D₂, E, C, B₆, PP.

The K1, K5, K10 strains used are phenol-resistant, possess an increased antibiotic activity with respect to bacteria of the Escherichia coli group including pathogenic microorganisms. Their physiological appropriation to an infant's organism is of great importance. ("Producing Child's Milk Products", Moscow, Legkaya i Pischevaya Promyshlennost PH, 1982, p.67 (in Russian).

As to its composition, such a known fermented milk is characterized by the content of the following ingredients: protein, 1.8 - 1.9 wt.%; fats, 3.5 wt.%; carbohydrates, 7.0 wt.%; mineral substances, 0.5 wt.%; water, 87.1 - 87.2 wt.%; vitamins, wt.%: A - 0.06; D₂ - 0.01; E - 10.0; C - 5.0; B₁ - 0.05; B₂ - 0.07; B₆ - 0.04; PP - 0.4.

An acidity of the known product is 50 - 70 ^{o}T, from 10⁷ to 10⁸ bacteria of Lactobacterium acidophilum species are contained in 1 g of the product.

An energy value of the product is 65 kcal (per 100 g of the product).

During vital activity of microorganisms of Lactobacillum acidophilum species, strains K1, K5, K10, partial proteolysis of milk proteins to peptides and free amino acids takes place. The content of essential amino acids of the fermented milk protein is as follows, wt.%: lysine, 7.0; threonine, 2.6; methionine, 0.9; valine, 5.5; phenylalanine, 4.4; leucine, 14.8; isoleucine, 3.2; histidine, 2.5; tryptophan, 2.1. ("Use of Acidophilic Mixtures for Feeding Healthy and Sick Infants in the 1st Year of Life", Moscow, 1980, p. 4 - 7 (in Russian).

In spite of the fact that the abovesaid fermented milk is balanced with respect to its protein, fatty-acid, carbohydrate, vitamin and mineral content which positively influences the intestine biocenosis, child's immune reactivity, vitamins constituting the product are not produced by microorganisms used in the process of milk fermentation but are introduced, that is, they are artificial substances.

Known in the art presently is production of fermented milk products possessing high dietary properties, easy assimilability, curative properties, a high caloric value by means of Lactobacterium acidophilum strain N.V. Ep 317/402. When preparing a fermented milk product, 1.0 wt % of strain is introduced into a whole milk at 28 - 40 ^{o}C, wherein milk clotting occurs within 5-8 hours (SU, A, 1,635,573).

The strain used possesses a slow acid-forming capability, its maximum acidification value reaching 360 ^{o}T.

Bacteria of said strain are capable of producing a substantial amount of antibiotics harmless to humans and especiallly to infants, which suppresses a growth and development of both Gram-positive and Gram-negative bacteria including all pathogenic bacteria causative of active forms of gastrointestinal diseases, the growth suppression zones being as follows: Staphilococcus aureus, 30 - 35 mm, Escherichia coli, 20 - 25 mm, Salmonella typhimurium, 20 - 23 mm, Klebsiella 20 - 22 mm.

A 36-year period of investigations and research work has shown the absence of phagolysis with said strain.

The abovesaid Lactobacterium acidophilum strain N.V. 317/402 was produced in 1949 by a directional selection in Yerevan (The Armenian Republic). A stock culture was isolated from a healthy human organism - from feces of newborns.

### Methods for the strain storage

- on a liquid nutrient medium at +5 to +8 ^{o}C
- a lyophilized culture at +5 to +8 ^{o}C
- on liquid nitrogen at -193 ^{o}C.

Growth conditions: facultative anaerobe.
Reinoculation frequency: every 1 - 1.5 months.

### Cultural and morphological features

Homofermentative, immobile, Gram-positive, microaerophilic, asporogenic bacilli measuring 2-20 * 0.8-0.9 µm. Cells are disposed singly or in short chains. Colonies on elective agar nutrient media are subsurface shallow, most commonly form balls consisting of entangled threads (R-forms).

### Physiological, biochemical, and genetic features

The strain does not reduce nitrates. Does not form skatole and indole. Does not dilute gelatine. Does not possess a hemolytic activity. Capable of fermenting the following substances: lactose, glucose, sucrose, fructose, mannose, levulose, galactose, maltose, raffinose, starch, dextrin, sorbitol, mannitol, dulcitol. Does not ferment inositol. Phenol resistance in milk is 0.9 - 1.0 %, synthomycin resistance is 0.003 %.

In the process of culturing, the strain enhances the vitamins content of milk: folic acid by 60 - 66 %, thiamine by 50 - 72 %, riboflavin by 11 - 32 %.

The product obtained by souring a milk in the presence of the abovesaid microorganisms of the Lactobacterium acidophilum N.V. strain 317/402 at an acidity of 60 - 90 ^{o}T fails to allow humans, while consuming the product, to receive the entire complex of amino acids required and hence it cannot be used as a complete breast milk substitute.

One more process for producing a fermented-milk product is known to comprise milk processing at 120 ^{o}C for sterilization, cooling milk to 30 - 34 ^{o}C and adding thereto from 0.2 to 0.4 wt% (of the milk weight) of the Lactobacterium acidophilum strains NK₁, K₃III₂₄, 100 AIII taken in the 2:2:1 ratio, respectively. Culturing is carried out at 30 - 34 ^{o}C for 18 - 24 hours to sour the milk up to a 180 - 210 ^{o}T acidity (SU, A, 1,004,471).

The product thus obtained can be used as a bacterial preparation - a starter with high selection properties for producing dietary and curative food products. In addition, the fermented milk product obtained can be directly taken in as food.

The resultant fermented milk product is characterized by an antagonistic activity against Shigella sonnei 99.9 %, Shigella flexneri, 100 %, Salmonellae, 99.3 %, Proteus spp., 99.9 %, and possesses a proteolytic activity of 62.5 %. The product is resistant to monocycline, neomycine, kanamycin, polymixin, penicillin, erythromycin, streptomycin, and tetracycline.

Moreover, the product obtained possesses a resistance to drying over the range of 45 - 58 % of viable cells on freeze drying, a storage stability over the range of 80 - 90 % of viable cells in the dry form storage at room temperature.

However, the known process fails to provide production of products used for treating acute forms of gastrointestinal diseases, premature infants, and adult patients of all age brackets.

### Disclosure of the Invention

The present invention has for its principal object to provide a process for producing a biologically active product "Acidolact-Narineh" and by imparting curative and nutritive properties to said target product providing conditions for formation of a complex of vitamins B, PP, E and broad-spectrum antibiotics physiological for a human organism.

The foregoing object is accomplished due to the fact that a biologically active fermented-milk product "Acidolact-Narineh", comprising proteins, fats, carbohydrates, organic acids, vitamins, water, and lactic acid fermentation microorganisms of Lactobacterium acidophilum species according to the present invention, contains symbiotic microorganisms of at least one of the group Ep-2 strains with the N.V. Ep 317/402 strain taken in a 1:1-4 ratio, respectively, in an amount of 10⁶-10⁹ symbiotic microorganisms per gram of the product, as lactic acid fermentation microorganisms of Lactobacterium acidophilum species, wherein said ingredients are contained in the following amount:

| | |
|---|---|
| proteins | 0.35 - 34.0 wt. % |
| fats | 0.05 - 36.0 wt. % |
| carbohydrates | 1.5 - 70.0 wt. % |
| organic acids (in terms of lactic acid) | 0.6 - 56.0 wt. % |
| vitamins per 100 g | 0.95 - 15.9 mg |
| water | 3.7 - 94.6 wt. % |

wherein the product has an acidity of 60 - 280 ^{o}T, and an energy value of from 20 to 540 kcal per 100 grams of the product.

The proposed product is suitable as a food product, in particular for feeding infants from the first days of life instead of a breast milk and, also, may be used as a drug in acute and severe forms of gastrointestinal tract diseases caused by Escherichia coli, Enterobacter 133, salmonellosis-groups of infections, Proteus sp., hemolytic jaundice, in diseases caused by Staphylococcus aureus and an opportunistic-pathogenic group of bacteria (Klebsiella and Pseudomonas aeruginosa).

According to the present invention, the herein-proposed "Acidolact-Narineh" acquires stronger bactericidal properties if it additionally contains microorganisms of Lactobacillus salivarius species, for example, the 1588 strain.

According to the proposed invention, the fermented-milk product "Acidolact-Narineh" contains proteins in an amount of 1.8 - 4.0 wt.%, fats - 0.05 - 6.0 wt.%, carbohydrates - 1.5 - 6.4 wt.%, organic acids (in terms of lactic acid) - 0.6 - 3.2 wt.%, vitamins in an amount of 0.95-1.59 mg per 100 g, water - 84.2 - 91.90 wt.%.

The fermented-milk product "Acidolact-Narineh" of the aforesaid composition has an acidity of 60 - 280 ^{o}T and may be used as a food product for infants over the age of three months, and adults. Furthermore, the product with such a composition may be used as a milk souring starter, as well as a treatment-and-prevention agent in diseases of the gastrointestinal tract.

An embodiment of the proposed invention consists in that fermented-milk product "Acidolact-Narineh" contains proteins in an amount of 0.35 - 0.8 wt.%, fats - 0.05 - 0.20 wt.%, carbohydrates - 1.5 - 3.8 wt.%, organic acids (in terms of lactic acid) - 0.7 - 3.15 wt.%, vitamins in an amount of 1.18 - 1.54 mg per 100 g, water - 93.20 - 94.6 wt.%, which permits its use for feeding sick premature infants and treating acute and severe forms of diseases of the gastrointestinal tract.

According to the proposed invention, it is expedient that the protein components of the product "Acidolact-Narineh" contain the following essential amino acids, in percent of the sum of amino acids in the product: valine (10.4 - 10.77), isoleucine (9.6 - 9.7), leucine (13.6 - 14.1), lysine (3.6 - 5.0), methionine (2.7 - 3.1), threonine (0.9 - 1.1), tryptophan (0.5 - - 0.7), phenylalanine (8.4 - 8.5).

According to the proposed invention, the product "Acidolact-Narineh" may contain the following vitamins per 100 g of the product:

| | |
|---|---|
| Vitamin A, traces | 0.050 mg |
| Pantothenic acid | 0.10 - 0.190 mg |
| Vitamin C | 0.5 - 0.90 mg |
| Biotin | 0.02 mg |
| Vitamin E (tocopherol), traces | 0.2 mg |
| Nicotinic acid | 0.040 - 0.047 mg |
| Vitamin PP (niacin) | 0.09 - 0.14 mg |
| Vitamins of the B group | 0.218 - 0.315 mg |
| including: | |
| Vitamin B₁ (thiamine) | 0.04 - 0.07 mg |
| Vitamin B₂ (riboflavin) | 0.15 - 0.20 mg |
| Vitamin B₆ (pyridoxine) | 0.025 - 0.04 mg |
| Vitamin B_{c} (folio acid) | 0.003 - 0.005 mg |

According to the proposed invention, the product "Acidolact-Narineh" may have the following components:

| | |
|---|---|
| proteins | 24.0 - 34.0 wt. % |
| fats | 0.6 - 36.0 wt. % |
| carbohydrates | 20.8 - 50.5 wt. % |
| organic acids (in terms of lactic acid) | 4.4 - 36.4 wt. % |
| water | 3.7 - 4.0 wt. % |
| vitamins per 100 g of the product | 9.5 - 15.9 wt. % |

The product of the aforesaid composition withstands a long-term storage without deterioration of its quality.

According to the proposed invention, the composition of "Acidolact-Narine" may be as follows:

| | |
|---|---|
| proteins | 6.0 - 12.9 wt. % |
| fats | 0.9 - 3.3 wt. % |
| carbohydrates | 26.7 - 70.2 wt. % |
| organic acids (in terms of lactic acid) | 15.4 - 56.0 wt. % |
| vitamins per 100 g of the product | 11.8 - 15.4 wt. % |
| water | 4.0 wt. % |

According to the proposed invention, it is expedient to produce the abovesaid fermented-milk product "Acidolact-Narineh" using the process comprising heat-treating the milk at 95 - 140 ^{o}C, cooling it to a souring temperature, adding microorganisms of Lactobacterium acidophilum species, and souring the milk to produce the desired product; at least one known strain of the Ep-2 group is used with the N.V. Ep 317/402 strain as microorganisms of Lactobacterium acidophilum species, wherein the Ep-2 strain is used in an amount that provides a 1:1-4 ratio to the amount of the N.V. Ep 317/402 strain, and souring is carried out until an 60 - 280 ^{o}T acidity is attained.

It is due to the proposed invention that it has become possible to obtain a product possessing nutritive, preventive and curative properties due to the presence of vitamins B₁, B₂, B₃, B₆, B_{c}, PP, and E in the finished product, as well as antimicrobial substances of the steroidal type which are physiologically appropriate for infants.

According to the proposed invention, it is expedient that the milk being soured be doped with microorganisms of Lactobaccillus salivarius species taken in an amount that provides a 1:1 ratio to said microorganisms of Lactobacterium acidophilum species, which makes it possible to improve bactericidal properties of the product obtained, to extend the range of its additional applications in veterinary medicine.

### Best Method of Carrying Out the Invention

An embodiment of the proposed invention consists in that a soured product having a 60 - 280 ^{o}T acidity is additionally freeze-dried to a moisture content of not more than 4 wt.%, which makes it possible to obtain a product suitable for storage under elevated temperatures without affecting the biochemical properties thereof.

Further objects and advantages of the present invention will be obvious from the following detailed description of "Acidolact-Narineh" (which hereinafter is referred to as Acidolact for the sake of brevity), the process for producing this fermented-milk product and specific examples of producing the proposed product and the process for producing same.

According to the present invention, there is provided a biologically active fermented-milk product Acidolact, containing mainly the following ingredients:

| | |
|---|---|
| proteins | 0.35 - 34.0 wt. % |
| fats | 0.05 - 36.0 wt. % |
| carbohydrates | 1.5 - 70.0 wt. % |
| organic acids (in terms of lactic acid) | 0.6 - 56.0 wt. % |
| water | 4.0 - 94.6 wt. % |

vitamins of group B, vitamin C, pantothenic acid, biotin, vitamin E (tocopherol), nicotinic acid, vitamin PP in an amount of 0.95 - 15.9 mg in 100 grams of the product, as well as symbiotic microorganisms of Lactobacterium acidophilum species of at least one of the group Ep-2 strains with the N.V. Ep 317/402 strain in an amount of 10⁶ - 10⁹ per gram of the proposed product.

Apart from the above-said ingredients, a liquid product contains mineral substances taken in the following amounts per 100 g of the initial product: sodium, 50 mg; potassium, 146 mg; calcium, 121 mg; magnesium, 14 mg; phosphorus, 90 mg; iron, 0.1 mg.

Acidolact obtained according to the present invention, can be obtained from milk, for example, cow milk, goat milk, buffalo milk, which is first of all subjected to heat treatment, the temperature of which depends upon climate conditions of the milk processing, the purpose of product being produced, and the age of a consumer. Thus, for example, in producing acidolact aimed at using as a breast-milk substitute, heat treatment is carried out at 136 - 140 ^{o}C for 5 - 6 seconds, whereas when producing acidolact oriented to people of the remaining age brackets, milk is subjected to heat treatment at 80 - 95 ^{o}C for 5 - 7 minutes. In producing a fermented-milk product used as a sour or for medical purposes, a raw milk processing temperature ranges between 120 and 121 ^{o}C with a 19-20-minute exposure at said temperature.

Then, the heat-treated, that is, a sterilized milk is cooled to a temperature at which a directional vital activity of the inoculated lactic-acid cultures is possible, i.e., to 28 - 40 ^{o}C.

According to the present invention, symbiosis of the known Lactobacterium acidophilum strains of the Ep-2 group taken either separately or in any combination is used with the known Lactobacterium acidophilum strain N.V. Ep 317/402 as lactic-acid cultures of microorganisms. Here, according to the invention, the strains of the group Ep-2 are used in an amount providing a 1:1-4 ratio to the amount of the N.V. Ep 317/402 strain.

Whenever the aforesaid ratio is not observed, the product obtained features a rather watery consistency and deviations in the chemical composition, which affects nutritive and curative properties of the product.

Due to fermentation in the presence of the proposed microorganisms, it has become possible to obtain the product with a rich amino acid composition prevailing towards an increased content of essential amino acids both in percentage ratio to protein and in absolute magnitude thereof.

The proposed symbiosis of known microorganisms is easily adaptable in the gastrointestinal tract of humans and animals. It is also adaptable to high phenol concentrations of milk, as well as to toxic chemicals, chemopharmaceuticals, and antibiotics; the aforesaid symbiosis of microorganisms contributes to the treatment of the diseases caused by Gram-positive and Gram-negative bacteria, pathogens of acute forms of gastrointestinal diseases, because of its being an active antagonist of opportunistic-pathogenic and pathogenic microorganisms.

Due to a lactic-acid fermentation of milk in the presence of symbiosis of the preselected abovenamed microorganisms, the product obtained is capable of suppressing the growth and development of putrid and pathogenic microorganisms in the gastrointestinal tract, wherein toxic metabolites are destroyed, and vitamins B₁, B₂, B₃, B₆, B_{c}, PP and E as well as harmless nontoxic broad-spectrum antibiotics physiologically appropriate for child's organism are synthesized.

As our studies have shown, the fermented-milk product Aoidolact containing the proposed symbiosis of microorganisms is an inducer of α- and γ-interferons and is able to improve activity of the interferon-NK system.

According to the present invention, it is expedient that the fermented-milk product of the invention be doped with the known microorganisms of Lactobaccillus salivarius species, for example, the 1588 strain, in an amount providing a 1:1 ratio to said microorganisms of Lactobacterium acidophilum species which makes it possible to add to the bactericidal properties of the product obtained.

Bacteria of this species are Gram-positive, homofermentative, immobile, granular and nongranular, asporogenic ones. Cells are disposed singly, or in short or long chains, they do not reduce nitrates and do not form indole, skatole nor dilute gelatine. The bacteria do not possess a hemolytic activity. They capable of fermenting the following substances: galactose, lactose, maltose, mannitol, sucrose, esculin, melibiose, raffinose, rhamnose, sorbitol. A limiting acid formation is 230 - 260 ^{o}T. Do not form gas from glucose, do not grow at 15 ^{o}C. An optimum cultivation temperture is +39 or +40 ^{o}C.

Acidolact prepared under the said conditions has an acidity of 60 - 280 ^{o}T. To produce a product suitable for feeding and treating infants in case of acute and severe forms of gastrointestinal-tract dieseases, it is proposed to carry cut souring up to an acidity of 60 - 165 ^{o}T.

In order to produce a concentrated product suitable for medical applications in vitro and in vivo, it is proposed to carry out souring up to an acidity of 270 - 280 ^{o}C.

Acidolact produced using the proposed process contains symbiotic microorganisms of Lactobacterium acidophilum species of at least one strain from the Ep-2 group with the N.V. Ep 317/402 strain and, probably, microorganisms of Lactobacillus salivarius species, the 1588 strain. These microorganisms are contained in an amount of 10⁶ - 10⁹ per gram of the product.

As to its composition, Acidolact produced as a result of fermentation under the above conditions, contains proteins in an amount of 1.8 - 4.0 wt.%, fats - 0.05 - 6.0 wt.%, carbohydrates - 1.5 - 6.4 wt.%, organic acids (in terms of lactic acid) - 0.6 - 3.2 wt.%; 100 g of the product contain: vitamin A in an amount of from traces to 0.05 mg, vitamins of group B, from 0.2180 to 0.3150 mg, namely, vitamin B₁ (thiamine) - 0.04 - 0.14 mg, vitamin B₂ (riboflavin), 0.15 - 0.2 mg, vitamin B₆ (pyridoxine), 0.025 - 0.040 mg, vitamin B_{c} (folic acid), 0.003 - 0.005 mg; vitamin C in an amount of 0.05 - 1.0 mg, pantothenic acid, from 0.100 to 0.2 mg, biotin, 0.002 mg, vitamin E (tocopherol) in an amount of from traces to 0.2 mg, nicotinic acid, from 0.040 to 0.047 mg, vitamin PP in an amount of 0.09 to 0.14 mg, water in an amount of from 84.2 to 91.9 wt.%, wherein the content of essential amino acids is as follows, in percent of the sum of amino acids in the product:

| | |
|---|---|
| valine | 10.4 - 10.77 |
| isoleucine | 9.6 - 9.7 |
| leucine | 13.6 - 14.1 |
| lysine | 3.6 - 5.0 |
| methionine | 2.7 - 3.1 |
| threonine | 0.9 - 1.1 |
| tryptophan | 0.5 - 0.7 |
| phenylalanine | 8.4 - 8.5 |

while the content of nonessential amino acids can be as follows, in percent of the sum of amino acids in the product:

| | |
|---|---|
| alanine | 4.10 - 4.15 |
| arginine | 6.1 - 6.6 |
| aspartic acid | 2.4 - 2.9 |
| histidine | 1.4 - 2.8 |
| cystine | 0.2 |
| glutamic acid | 5.7 - 6.2 |
| proline | 11.2 - 11.8 |
| serine | 2.7 - 2.9 |
| tyrosine | 11.0 - 11.1 |
| glycine | 1.4 - 2.1. |

The present invention provides for a possibility of additional freeze-drying of a soured product with an acidity of 60 - 280 ^{o}T until a moisture content of not more than 4 wt.% is attained, which makes it possible to produce a product suitable for a long-term storage under elevated temperatures, with the biochemical properties thereof remaining unaffected, wherein the proposed product has the following composition as to its main ingredients:

| | |
|---|---|
| proteins | 24.0 - 34.0 wt. % |
| fats | 0.6 - 36.0 wt. % |
| carbohydrates | 20.8 - 50.0 wt. % |
| organic acids (in terms of lactic acid) | 4.4 - 36.4 wt. % |
| vitamins per 100 grams | 9.5 - 15.9 mg |
| water | 3.7 - 4.0 wt. % |

A moisture content of the dried product above 4 wt.% fails to provide for preservation of quality of the end product.

According to the present invention, it is expedient that prior to drying the soured product, be separated, under aseptic conditions, into a liquid and a protein fraction, each of them can subsequently be dried separately, which allows of obtaining a highly antibacterial concentrate suitable for use in medical purposes.

To produce Acidolact suitable for feeding and treating premature infants and children of all ages, it is therefore necessary to separate the soured product under aseptic conditions to obtain a liquid fraction which is in fact a fermented-milk product having substantially the following composition:

| | |
|---|---|
| proteins | 0.35 - 0.8 wt. % |
| fats | 0.05 - 0.20 wt. % |
| carbohydrates | 1.5 - 3.8 wt. % |
| organic acids (in terms of lactic acid) | 0.70 - 3.15 wt. % |
| vitamins | 1.18 - 1.54 mg/100 g |
| water | 3.7 - 94.6 wt. %. |

The protein fraction remaining upon separation of the liquid fraction, has substantially the following composition:

| | |
|---|---|
| proteins | 5.5 - 11.0 wt. % |
| fats | 0.1 - 6.0 wt. % |
| carbohydrates | 1.6 - 2.0 wt. % |
| organic acids (in terms of lactic acid) | 1.0 - 2.0 wt. % |
| vitamins | 1.2 - 1.56 mg/100 g |
| water | 83.4 - 86.6 wt. % |

The proposed invention allows of producing the product aimed at using for curative purposes in gynecology, pediatrics and for treating patients with diseases of the gastrointestinal tract. To this end, the abovesaid liquid fraction separated under aseptic conditions is freeze-dried up to a maximum moisture content of 4 wt.% to obtain a powdered substance with the following composition:

| | |
|---|---|
| proteins | 7.7 - 12.9 wt. % |
| fats | 0.8 - 3.3 wt. % |
| carbohydrates | 26.7 - 70.2 wt. % |
| organic acids (in terms of lactic acid) | 15.4 - 56.0 wt. % |
| vitamins per 100 grams | 11.80 - 15.4 mg |
| water | 4.0 wt. %. |

The resultant fermented-milk product Acidolact is nontoxic, produces no carcinogenic, mutagenic, or allergenic effect, is an inducer of an increased number of α- and γ-interferons in the organism and a stimulant of the interferon-NK system.

The obtained liquid fermented-milk product Acidolact is a moderately thick, slightly tough mass having a proper fermented-milk odor and taste.

The composition of the product obtained is proved by means of chemical and microbiological analyses.

The proposed fermented-milk product Acidolact shows such properties that permit its use for treating acute severe forms of gastrointestinal diseases (dysbacteriosis, salmonellosis, dysentery, common diarrheas) as well as hemolytic jaundice, omphalitis in newborn, in obstetric-and-gynecological practice for treating vulvovaginitis, and other pyo-inflammatory diseases; for treating urethritis, allergies, and hemo-, hepato-, and renopathies, also atrophia and dystrophia in children.

The proposed product Acidolact contains an increased amount of protein and is therefore recommended for use in the diet of people exposed to radioactive irradiation, since proteins promote a more rapid removal radioactive nuclides from the organism.

Therapeutic properties of the proposed product owe their origin to accumulated metabolites of lactic-acid cultures therein. In addition to the preparation of steroidal antibiotics, vitamins B₁, B₂, B₃, B₆, B_{c}, PP, and E which are physiologically appropriate for a child's organism are produced. During storage, a further accumulation of antibiotics takes place, which promotes an increase in a storage stability of the proposed product.

Many-year experimental testing of the proposed process for producing the fermented-milk product "Acidolact-Narineh" has shown the product to feature stability in its production with constant quality characteristics as to acidity, amino acid composition, and vitamin content.

The proposed fermented milk has been tested experimentally on animals and trialled under clinical conditions to study its activity in treating the above diseases.

The herein-proposed product was tested for effect on the course of a wound healing process under conditions of its local use.

Studies were performed on 24 chinchilla rabbits.

Standard skin-fascial wounds measuring about 2 * 2.5 cm (500 sq. mm) in size were inflicted on the back area of all the test rabbits; a skin graft was dissected with subcutaneous fat and fascia up to a muscular layer, a wound lips with a subcutaneous muscle were crushed with Kocher's forceps. Once hemostasis had occurred, rabbits were infected with 1 ml of a microbial suspension, containing 1.5 * 10⁹ microbial bodies of a polyresistant hospital strain of Staphylococcus aureus injected into the wound. After 48 hours postinfection, a pronounced purulent inflammation was revealed in the test animals.

All the animals were subdivided into three groups each containing eight rabbits. In Group I (test), animals were treated with the proposed fermented-milk product. For the purposes of comparison, rabbits of Group II were treated with Rivanol. Group III (control) consisted of untreated animals. The treatment was started on the third day postinfection. The afore mentioned drugs were applied to the wound locally in the form of liberally wetted, loosely placed tampons and applications, whereupon aseptic dressings were applied thereto. Animals of the control group were kept under similar conditions, being also subjected to every day redressing with a wound assanitation and application of aseptic dressings.

Clinical course of a wound healing process was controlled based on a number of criteria such as the nature and amount of the discharge, terms for arresting a perifocal inflammatory reaction, necrolysis terms, emergence of granulations, epithelization, terms of a wound healing. Parallel with this, the area of a wound surface was measured, cytological and morphological studies of the biopsy specimens taken from the wound edge and bottom, bacteriological studies of changes in a wound discharge (prior to and during the treatment) were carried out. To determine the presence of microbes in sections, the specimens were Gram-Weigart stained. Bacteriological studies were carried out with regard to a rate and duration of staphylococci dissemination from an affection focus. The following quantitative characteristics of dissemination were used: up to 25 colonies on a Petri dish - a single growth; up to 70 colonies - a poor growth; up to 200 colonies - an abundant growth; more than 200 colonies - an entire growth. A material from a purulent wound was taken with a calcined and quenched standard loop, 3 mm in diameter, emulsified in 1 ml saline and then plated by 0.05 ml onto Petri dishes with the plain agar. The plated material was incubated in a thermostat at 37 ^{o}C for 18 - 20 hours followed by counting staphylococcus colonies.

When comparing the course of a wound healing process in all the three groups of animals as to the main clinical findings (see Table I), the processes of necrolysis, emergence of first granulations and epithelization, arresting of the inflammatory reaction in a wound were found to occur in the test rabbits of Group I much earlier than in those of Groups II and III, a complete wound healing in said Group I proceeded relatively faster (on the average, twice as fast as in the control groups).

The analysis of the results of bacteriological studies of a purulent wound discharge in rabbits of the control and of the two tested groups treated with Rivanol and the proposed fermented-milk product, showed a high antibacterial efficiency of the latter product.

Histological and histochemical studies of a progress in a wound healing on the 5th day of treatment showed that there was a pronounced bacterial contamination of wounds against a background of drastic inflammatory changes in deep layers in the animals of the control group.

On the 10th day, inflammatory changes with an abundant microflora were revealed in the wound surface and deep layers. There were foci of necrosis, microabscesses in the subcutaneous fat, a purulent skin infiltration encompassing areas of the conserved epidermis and subcutaneous fat of the wound edges.

On the 15th day, compared to the preceding period, a relative weakening the intensity of an inflammatory process and thinning of the granular-fibrinous layer on the wound surface were observed. A thin layer of a new-growth epithelium from the wound edges creeped over a granulation tissue. A pronounced purulent infiltration involving necrotic changes and the emergence of purulent fusion foci (microabscesses) were revealed in the surface layers of the granulation tissue, as well as in the subjacent tissues. Round-cell infiltrates of mainly a lymphophagocytic nature, single macrophages, phagocyte leukocytes were formed around a pronounced capillary network.

The use of the proposed fermented milk greatly accelerates reparative processes in wounds: thus, by the 5th day of treatment, a marked thinning of a granular-fibrinous layer was observed with a decrease in the number of stab neutrophile leukocytes and an arrest of an inflammaroty process in tissues adjacent to the wound. A comparative cleaning of the wound cavity from necrotyc areas and fibrinopurulent impositions was observable with the prevalence of a serous component in the pus mass. A considerable expansion of a granulation tissue was also observable.

On the 10-15th day, reparative processes in the wound were expressed far stronger over the control. Thus, by the 15th day, in a majority of cases a complete reconstitution of an epithelial coat on the wound surface was observable with a tendency to thickening and recovering all the layers thereof. A surface layer of the granulation tissue was represented by a fine-fibrous connective tissue with a papillary excrescence, whereas a deep layer was represented by a coarse-fibered fibrous tissue. A differentiation of vessels into arterioles and venules took place.

The study of cicatrices of animals in all three groups showed that those in Group I were characterized by a uniform epithelial lining with the reconstitution of all layers thereof, and in deep layers, by an ordered disposition of thin, gentle collagenic fibres the bundles of which had a horizontal arrangement. In the animals of Group II, a coarser cicatricial tissue prevailed, in some places with the absence of the epithelial coat and a more disordered disposition of collagenic fibres in the fibrous connective tissue. In rabbits of Group III, a cicatrix was presented by a coarse fibrous tissue on the surface of which elements of skin were absent, connective-tissue fibres in deeper layers had a disordered disposition, phenomena of sclerosis, single foci of a chronic infection in the form of granulomas with sporadic staphylococci took places.

Thus, all mentioned above bears witness to the fact that metabolites of lactic acid bacteria of the proposed symbiosis, when applied locally to the induced models of purulent wounds under experimental conditions in test rabbits, contribute to a faster wound cleaning from necrotic tissues and pyogenic microflora, stimulate the regeneration processes in wounds, shorten the periods of a wound healing, thus promoting a wound healing with a soft, elastic cicatrix.

**Table 1**

| | Number of animals | First granulation advent, days | Full wound healing, days | Edge epitelization advent, days | Full wound filling with granules, days | Full wound healing days |
|---|---|---|---|---|---|---|
| Proposed acidolact | 8 | 3.375 | 6.1 | 7.5 | 10.5 | 14.6 |
| Rivanol | 8 | 7.0 | 13.5 | 15.1 | 18.9 | 24.7 |
| Control | 8 | 8.9 | 17.5 | 19.6 | 23.0 | 29.0 |

The data obtained show the expediency of a clinical trialling and approvement of the proposed fermented-milk product.

The proposed fermented-milk product was used with 1427 sick and healthy carriers of pathogenic microorganisms. There were no complications associated with its application, which indirectly points to the fact that the creation of artificial biocenoses with the assistance of lactic-acid bacteria does not result in the emergence of their virulent mutants capable of causing a purulent pathology.

Patient suffered from pyo-inflammatory diseases of the urinary bladder and urethra was treated with the proposed fermented-milk product having an acidity of 165 ^{o}T.

The treatment procedure was as follows: The fermented-milk product Acidolact containing not more than 0.1 wt.% of fat, according to the present invention, was introduced into the cavity of the urinary bladder (via Pezzer's or Nelaton's catheter), as wall as directly into urethra. Exposure time was 60 - 120 minutes, the product temperature, 37 - 40 ^{o}C. Patients with suprapubic vesical fistulas were given the proposed product in an amount of 70 - 100 ml for 10 - 12 days; patients with cystitis, 100 - 150 ml for 7 - 10 days; patients with urethritis, 10 - 15 ml for 8 - 10 days.

A total of 85 patients were treated by said method. The control group consisted of 50 patients treated by conventional medicinal methods. Patients treated with the proposed product exhibited a number of obvious advantages: improvement in their general condition, delitescence of painful sensation, pronounced reduction of dysuric effects, normalization of body temperature, ceasing of pyorrhea, and improvement in the wound granulation.

These changes usually approached on the 9th day of treatment. Laboratory studies revealed a shift in urine pH from alkaline to acidic (urine pH reduced from 8 to 7 with 62 % of patients of the control group and in the case of using Acidolact, pH reduced from 8 to 5.4 with 87 % patients /P<0.01/). Leukocyturia decreased markedly (2.4 times in 57 % of patients of the control group, and 5.0 times in 23 % of patients of the test group /P<0.01/). Decrease in proteinuria was also observed /P<0.02/. Bacteriuria, when treated conventionally, reduced from 5 - 10 million to 500.000 - 1.000.000 microbial cells in 1 ml of urine in 53 % of patients. Following treatment with the claimed product, bacteriuria reduced to 5.000 - 50.000 in 76 % of patients /P<0.02/. There was no evidence of a growth of pathogenic microorganisms in 15 % of patients.

During a three-month period (once a month), the examined carriers of a strepto-staphylococcal infection were subjected to a bacteriological study and remained under observation during a year (once every three months, a bacteriological study of the nasopharyngeal ring was performed).

Carriers of a strepto-staphylococcal infection were divided into two groups, one of which was subjected to assanitation with antibiotics, while the other group of the carriers was subjected to assanitation with the proposed fermented-milk product Acidolact by the following procedure: intranasally, by means of individual pipettes at a dose of 0.5 ml three times a day for 5 days. Administration of Acidolact was followed by massaging two front nasal septa.

After a two-month observation period, as low as 0.8 % of the patients who had received the claimed product, remained infection carriers, whereas among those received antibiotics 2.2 % of the patients remained infection carriers.

The proposed fermented-milk product Acidolact was also used to normalize biocenosis in obstetrics and gynecology.

Treatment was given to 186 girls, 165 gynecological patients, 64 parturients and puerperants; 157 pregnants were sanitized for antepartal treatment.

A dose of 10 - 20 ml of Acidolact was administered per diem for 8 - 15 days. In a child's gynecology, a treatment course was repeated in a month. The treatment efficiency was monitored in progress with the aid of a complex of clinico-laboratory methods, visual observations, colposcopy, bacterioscopic and bacteriological studies.

A total of 172 samples from the vagina and 84 from the cervical canal were subjected to a bacteriological study.

In the process of a bacteriological study of the vaginal contents, 91.7 % of the gynecological patients exhibited both pathogenic and opportunistic-pathogenic microflora, as well as Candida fungi. In a majority of cases, microbes were disseminated in associations.

A course of colpitis in pregnants often proceeded asymptomatically, wherein two-thirds the pregnants displayed stage IV of purity of the vaginal flora and more than 2 - 3 kinds of microbe associations.

Every third of the examined patients (39.5 %) exhibited a combination of an opportunistic pathogenic microflora (pathogenic staphylococci, Proteus, some serotypes of Escherichia coli, and Candida fungi).

In the group of parturients with a prolonged (more than 24 hours) water-free period, upon bacterial examination, a massive dissemination in the cervical canal was found in 23 out of 28 parturients, an opportunistic-pathogenic and pathogenic microflora were isolated in 82.1 % cases, in particular, St. aureus (47.6 %). The analysis into the results of treating gynecological patients and pregnants, on the completion of the first treating course showed that in respect to all the treated, without an exception, there was indicated a considerable improvement in clinical manifestations, a substantial reduction of the number of leukocytes, a complete loss of fungi. Bacterial examinations of gynecological patients showed a drastic decrease (83.4 %) in the dissemination of an opportunistic-pathogenic and pathogenic microflora with a primary prevalence of rod-shaped (bacilliform) bacteria. Bacterioscopic studies of smears revealed a change in a vaginal flora purity with the transitions from the fourth to the second stage. Dissemination reduction in pregnants amounted to 75.8 %.

The obtained results of clinico-bacteriological stidies in respest to pregnants and parturients enables one to conclude that the use of the proposed liquid Acidolact (produced on the base on a fat-free or fatty milk) is expedient in the obstetric-and-gynecological practice.

The use of the proposed fermented-milk product with an acidity of 120 ^{o}T in a liquid form produces an excellent therapeutic effect in child's gynecology. Dropping the proposed product into the vagina of girls using individual pipettes (for 15 - 20 days) and introducing it into the area of posterior vault of the vagina of adult females in an amount of 10 - 20 ml for 8 - 15 days showed a considerable improvement of clinical manifestations, a drastic reduction of the number of leukocytes and microflora dissemination from 70.8 % prior to treatment to 34.2 % in the posttreatment period, as well as the loss of pathogenic microflora and normalization of vaginal biocenosis.

To promote understanding, given below are the following examples of specific embodiment of the present invention.

### Example 1

A total of 10 kg of a fat-free cow milk is sterilized at 120 ^{o}C for 20 minutes, cooled to a souring temperature, i.d. 40 ^{o}C, then 240 g of symbiotic microorganisms consisting of 80 g of the 317/392, 317/395, 317/396 strains of group Ep-2 Lactobacterium acidophilum taken in equal amounts, and 160 g of the N.V. Ep 317/402 strain of Lactobacterium acidophilum are introduced. Symbiotic microorganisms are thoroughly mixed with the fat-free milk and allowed to stand for 47 hours. The obtained fermented product is cooled to +8 ^{o}C and allowed to stand again for 12 hours. An acidity of a liquid finished product is 280 ^{o}T.

A composition of the finished product as to its main ingredients is as follows: water, 91.8 wt.%; dry substances, 8.2 wt.%; including: proteins, 2.8 wt.%; fats, 0.1 wt.%; carbohydrates, 1.5 wt.%; organic acids and other fermentation products, 3.1 wt.%; ashes, 0.7 wt.%; wherein 100 grams of the product contains the following elements: sodium, 52 mg; potassium, 146 mg; calcium, 121 mg; magnesium, 14 mg; phosphorus, 90 mg; iron, 0.1 mg; vitamins, 1.530 mg. An energy value is 29.2 kcal per 100 g of the product.

### Example 2

Fermented-milk product Acidolact produced under conditions similar to those described in Example 1 and having a composition similar to that indicated in Example 1, is freeze-dried at an initial temperature of minus 50 ^{o}C, a final temperature of minus 26 ^{o}C, and a residual pressure of 13.3*10³ Pa for 24 hours. The finished product has a moisture content of 3.7 wt.% and 96.3 wt.% of dry substances, and consists of the following main ingredients: proteins, 32.8 wt.%; fats, 1.1 wt.%; carbohydrates, 20.8 wt.%; organic acids, 36.4 wt.%; ashes, 5.2 wt.%; vitamins 15.40 mg/100 g, etc., the elementary composition of 100 g of the product is as follows: sodium, 501 mg; potassium, 1352 mg; calcium, 1120 mg; magnesium, 51 mg; phosphorus, 630 mg; iron, 1 mg. An energy value is 29.2 kcal per 100 g. The product contains 3*10⁹ microorganisms per gram.

The product obtained is used in surgical practice for treating pyo-inflammatory processes (topic application) or as a functional food for enlarging the activity of the interferon-NK system.

### Example 3

An amount of 10 kg of a fat-free cow milk is sterilized at 121 ^{o}C for 19 minutes and cooled to a 37 ^{o}C temperature; then 200 grams of symbiotic microorganisms consisting of 100 g of strain 317/392 of the group Ep-2 Lactobacterium acidophilum and 100 g of strain N.V. Ep 317/402 of Lactobacterium acidophilum are introduced. The symbiotic microorganisms are thoroughly mixed with a fat-free milk and allowed to stand at 37 ^{o}C for 5 hours. The fermented product obtained is cooled to +6 ^{o}C and allowed to stand again for 12 hours. An acidity of the liquid finished product is 76 ^{o}T.

The composition of the finished product as to the main ingredients is as follows: water, 91.5 wt.%; dry substances, 8.5 wt.%, including: proteins, 3.0 wt.%; fats, 0.05 wt.%; carbohydrates, 4.1 wt.%; organic acids, 0.65 wt.%; ashes, 0.7 wt.%; the elementary composition of 100 g of the product is as follows: sodium, 50 mg; potassium, 146 mg; calcium, 121 mg; magnesium, 14 mg; phosphorus, 90 mg; iron, 0.1 mg; vitamins, 1.950 mg. An energy value is 31.2 kcal per 100 g. The product is used as a sour and a dietary product.

### Example 4

To produce 1000 kg of the fermented-milk product Acidolact, 522 kg of a whole milk having the following fractional composition by weight: 3.2 % fats, 3.0 % proteins, 4.7 % carbohydrates, is doped with 6.23 kg of a refined olive oil premixed homogeneously with 63 kg of creams with 20 % by weight of fat , and 42.2 kg of carbohydrates (in terms of dry substances) of which 45 kg of dextrine-maltose containing 60 wt.% of carbohydrates and 15.2 kg of sugar. The mixture thus obtained is sterilized at 136 ^{o}C and cooled to 36 ^{o}C. Next 20 kg of Acidolact produced as in Example 3 is added to a cooled mixture and allowed to stand at said temperature for 5.5 hours, thereupon a soured mixture is cooled rapidly to +6 ^{o}C and allowed to stand for 12 hours. A homogeneous product with a delicate consistency is obtained, having a sweet fermented taste and an acidity of 67 ^{o}T.

The composition of the finished product as to the main ingredients is as follows: water, 87.05 wt.%; dry substances, 12.95 wt.%, including: proteins, 1.8 wt.%; fats, 3.5 wt.%; carbohydrates, 6.4 wt.%; organic acids, 0.6 wt.%. Mineral substances per 100 g of the product: sodium, 21 mg; potassium, 95 mg; calcium, 82 mg; magnesium, 7 mg; phosphorus, 50 mg; iron, 0.1 mg; vitamins, 1.23 mg. An energy value is 66.5 kcal per 100 g. One gram of the product contains 4*10⁸ cells of useful microorganisms.

The product thus obtained may be used as a breast milk substitute in a compound and artificial feeding of infants.

### Example 5

An amount of 1000 kg of a cow milk (3.5 wt.% of fats and 3.0 wt.% of proteins) is purified, heat treated at 95 ^{o}C, allowed to stand for 15 minutes and cooled to 39 ^{o}C. Then 30.0 kg of symbiotic microorganisms consisting of 15.0 kg of microorganisms of Lactobacterium acidophilum (5.0 kg of strains 317/392, 317/395, 317/396 of group Ep-2 and 10.0 kg of strain N.V. Ep 317/402) and 15.0 kg of microorganisms of Lactobacillus salivarius, strain No. 1588, are introduced. Symbiotic microorganisms are thoroughly mixed with the milk and allowed to stand at 39 ^{o}C for 3.5 hours. The obtained fermented product is cooled to +7 ^{o}C and again allowed to stand for 13 hours. An acidity of finished Acidolact is 155 ^{o}T.

The composition of the finished product as to the main ingredients is as follows: water, 88.1 wt.%; dry substances, 11.9 wt.%, including: proteins, 3.0 wt.%; fats, 3.5 wt.%; carbohydrates, 3.7 wt.%; organic acids, 1.0 wt.%; mineral substances per 100 g of the product: sodium, 50 mg; potassium, 146 mg; calcium, 121 mg; magnesium, 14 mg; phosphorus, 90 mg; iron, 0.1 mg; vitamins, 1.59 mg. An energy value is 62.0 kcal per 100 g. The number of microorganisms is 5*10⁹ per gram.

The product obtained may be used for oral administration in acute and severe forms of the diseases of the gastrointestinal tract.

### Example 6

An amount of 10 kg of a fat-free cow milk is purified, sterilized at 120 ^{o}C for 20 minutes, cooled to 36 ^{o}C, then 200 g of symbiotic microorganisms consisting of 100 g of strain 317/395 of the group Ep-2 Lactobacterium acidophilum and 100 g of strain N.V. Ep 317/402 of Lactobacterium acidophilum are introduced. The symbiotic microorganisms are thoroughly mixed with the milk and allowed to stand for 5.5 hours. The obtained fermented product is cooled to +6 ^{o}C and allowed to stand again for 12 hours. An acidity of the liquid fermented milk is 90 ^{o}T. It is used as a ferment for producing a breast milk substitute.

The composition of the finished product as to the main ingredients is as follows: water, 91.5 wt.%; dry substances, 8.5 wt.%, including: proteins, 3.0 wt.%; fats, 0.1 wt.%; carbohydrates, 3.9 wt.%; organic acids, 0.7 wt.%.

The following mineral substances are contained in 100 g of the product: sodium, 52 mg; potassium, 152 mg; calcium, 126 mg; magnesium, 14 mg; phosphorus, 95 mg; iron, 0.1 mg. 1.097 mg of vitamins are contained per 100 g of the product. An energy value is 31 kcal per 100 g. One gram of the product contains 5*10⁸ microorganisms.

### Example 7

Added to 10 kg of milk having the fat content of 2 wt.% and containing 3.0 wt.% of proteins, sterilized at 121 ^{o}C for 20 minutes, cooled to 38 ^{o}C, is 200 g of symbiotic microorganisms consisting of 50 g of microorganisms of strain 317/396 of the group Ep-2 Lactobacterium acidophilum and 150 g of microorganisms of strain N.V. Ep 317/402 of Laotobacterium acidophilum. The symbiotic microorganisms are thoroughly mixed with the milk and allowed to stand for 4.5 hours. The obtained fermented product is cooled to +8 ^{o}C and allowed to stand again for 12 hours. An acidity of the liquid fermented milk is 90 ^{o}T. The product obtained is separated under aseptic conditions into a liquid and a protein fraction.

The composition of the liquid fraction is as follows: water, 94.6 wt.%; dry substances, 5.4 wt.%, including: proteins, 0.35 wt.%; fats, 0.05 wt.%; carbohydrates, 3.7 wt.%; organic acids, 0.8 wt.%, ashes, 0.5 wt.%.

The following elements are contained in 100 g of the product: sodium, 41 mg, potassium, 125 mg; calcium, 60 mg; magnesium, 6 mg; phosphorus, 71 mg; iron, 0.1 mg; vitamins, 1.180 mg. An energy value is 19.6 kcal/100 g. One gram of the product contains 6*10⁶ useful microorganisms.

The liquid fraction is used as a dietotherapy for premature infants.

The composition of the protein fraction is as follows: water, 86.6 wt.%; dry substances, 13.4 wt.%, including: proteins, 5.5 wt.%; fats, 4.0 wt.%; carbohydrates, 2.0 wt.%; organic acids, 1.0 wt.%, ashes, 0.9 wt.%. The following elements are contained in 100 g of the product: sodium, 41 mg; potassium, 125 mg; calcium, 146 mg; magnesium, 23 mg; phosphorus, 100 mg; iron, 0.1 mg. An energy value is 19.6 kcal/100 grams. The content of vitamins is 1.32 mg per 100 g of the product. One gram of the product contains 6*10⁸ microorganisms.

The protein fraction with or without addition of carbohydrates is used as a food for infants over 6 months and patients of all age brackets.

### Example 8

The liquid fraction of the fermented-milk product produced under conditions similar to those described in Example 7, is freeze-dried at an initial temperature of minus 50 ^{o}C and a final temperature of minus 26 ^{o}C and a residual pressure of 13.3*10³ Pa for 23 hours. The obtained dried product retains its chemical and curative properties unaffected for two years. The finished product from the protein fraction contains: water, 4.0 wt.%; dry substances, 96 wt.%, including: organic acids, 12.6 wt.%; fats, 0.9 wt.%; proteins, 6.3 wt.%; carbohydrates, 70.6 wt.%/; ashes, 6.0 wt.%; 100 g of the product contain the following elements: sodium, 420 mg; potassium, 1300 mg; calcium, 600 mg; magnesium, 48 mg; phosphorus, 700 mg; iron, 1.5 mg. An energy value is 359 kcal per 100 g. One gram of the product contains 3*10⁶ microorganisms. The content of vitamins is 11.8 mg/100 g of the product.

The product obtained is used in medicine, as well as for feeding child and adult patients.

### Example 9

A total of 1000 kg of a sow milk containing 3.5 wt.% of fats and 3.0 wt.% of proteins is heat-treated at 95^{o}C, allowed to stand for 20 minutes and cooled to 39 ^{o}C. Then 20.0 kg of symbiotic microorganisms consisting of 4 kg of microorganisms of Lactobacterium acidophilum (strains 317/392, 317/395 of group Ep-2 taken in equal amounts) and 12 kg of microorganisms of strain N.V. Ep 317/402 are added to the milk. The symbiotic microorganisms are thoroughly mixed with the milk and allowed to stand at 38 ^{o}C for 4.5 hours. The obtained fermented product is cooled to +8 ^{o}C and allowed to stand again for 16 hours. An acidity of the finished product is 110 ^{o}T.

The product thus obtained is used for feeding infants from a three-month age on and patients of all age brackets affected by the diseases of the gastrointestinal tract.

The composition of the finished product as to the main ingredients is as follows: water, 88.1 wt.%; dry substances, 11.9 wt.%, including: proteins, 3.0 wt.%; fats, 3.5 wt.%; carbohydrates, 3.80 wt.%; lactic acid, 0.90 wt.%; ashes, 0.7 wt.%. The following elements are contained in 100 g of the product: sodium, 50 mg; potassium, 146 mg; calcium, 121 mg, magnesium, 14 mg; phosphorus, 80 mg; iron, 0.1 mg; vitamins, 1.54 mg. One gram of the product contains 6*10⁸ microorganisms. An energy value is 62.0 kcal per 100 g.

### Example 10

An amount of 10 kg of a fat-free cow milk is sterilized at 121 ^{o}C for 19 minutes, cooled to a 40 ^{o}C temperature, then there are added thereto 400 g of symbiotic microorganisms consisting of 200 g of equal parts of microorganisms of strain 317/401 of the group Ep-2 Lactobacterium acidophilum and strain N.V. Ep 317/402 of Lactobacterium acidophilum, taken in equal amounts, and 200 g of microorganisms of Lactobacillus salivarius, strain 1588. The mixture is let to stand still until a fermented clot is formed, cooled to 6 ^{o}C and allowed to stand for 12 hours. A chemical composition of the finished product primarily consists of: water, 91.75 wt.%; dry substances, 8.25 wt.%, including: proteins, 2.8 wt.%; fats, 0.1 wt.%; carbohydrates, 3.75 wt.%; lactic acids, 0.9 wt.%; ashes, 0.7 wt.%; 100 g of the product contains the following elements: sodium, 50 mg; potassium, 141 mg; calcium, 120 mg; magnesium, 15 mg; phosphorus, 80 mg; iron, 0.1 mg; vitamins, 1.27 mg. An energy value is 30.3 kcal per 100 g. One gram of the product contains 7*10⁸ microorganisms. The product is used as a sour or a functional food.

### Example 11

An amount of 80 kg of a fat-free milk is purified, sterilized at 120 ^{o}C for 20 minutes, cooled to a 40 ^{o}C temperature, then 2.0 kg of a ferment consisting of 0.5 kg of strain 317/392 of the group Ep-2 Lactobacterium acidophilum and 1.5 kg of strain N.V. Ep 317/402 of Lactobacterium acidophilum are added. Thereupon, the ferment is mixed with the milk and let to stand still for 4.5 hours. The fermented milk is then chilled quickly to +6 ^{o}C, allowed to stand for 20 hours and then packed into sterile 50-cu.cm containers. An acidity of the liquid fermented milk is 160 ^{o}T. It is used as a ferment and a medicinal agent for oral administration.

The composition of the finished product as to the main ingredients is as follows: water, 91.9 wt.%; dry substances, 8.1 wt.%, including: proteins, 2.8 wt.%; fats, 0.05 wt.%; carbohydrates, 3.6 wt.%; organic acids, 1.0 wt.%; ashes, 0.65 wt.%. 1.33 mg of the various vitamins is contained in 100 g of the product. An energy value is 29.6 kcal per 100 g. The content of mineral substances per 100 g is as follows: sodium, 49 mg; potassium, 142 mg; calcium, 125 mg; magnesium, 15 mg; phosphorus, 85 mg; iron, 0.1 mg. One gram of the product contains 6.0*10⁹ cells of microorganisms.

### Example 12

The product prepared in a way similar to that of Example 11 is freeze-dried at an initial temperature of minus 50 ^{o}C and a final temperature of minus 26 ^{o}C, and at a residual pressure of 13.3*10³ Pa for 24 hours. The main composition of the product is as follows: water, 4.0 wt.%; dry substances, 96 wt.%, including: proteins, 25.9 wt.%, fats, 0.6 wt.%; carbohydrates, 49.2 wt.%; organic acids, 13.5 wt.%; ashes, 6.8 wt.%; 100 g of the product contains the following elements: sodium, 501 mg; potassium, 1410 mg; calcium, 1230 mg; magnesium, 143 mg; phosphorus, 830 mg; iron, 0.5 mg; vitamins, 14.3 mg. An energy value is 354 kcal per 100 g. One gram of the product contains 3*10⁹ cells of microorganisms. The product obtained is used as a dry ferment for an industrially available product or as a medicinal agent for oral administration.

The product obtained retains its properties as a ferment unaffected for three months and as a medincinal agent, for two years.

### Example 13

An amount of 1000 kg of a cow milk (3.5 wt.% of fats and 3.0 wt.% of proteins) is filtered, heat-treated at 94 ^{o}C, allowed to stand for 15 minutes and cooled to 40 ^{o}C. Then there are added 30.0 kg of symbiotic microorganisms, consisting of 15.0 kg of microorganisms of Lactobacterium acidophilum, strain 317/396 of group Ep-2 and strain N.V. Ep 317/402 taken in a 1:4 ratio, respectively, and 15.0 kg of microorganisms of Lactobacillus salivarius, strain 1588. The symbiotic microorganisms are thoroughly mixed with the milk and allowed to stand at 40 ^{o}C for 4.0 hours. The obtained fermented product is cooled to +7 °C and allowed to stand again for 14 hours. An acidity of the finished Acidolact is 165 ^{o}T.

The composition of the finished product as to the main ingredients is as follows: water, 88.0 wt.%; dry substances, 12.0 wt.%, including: proteins, 3.1 wt.%; fats, 3.5 wt.%; carbohydrates, 3.7 wt.%; organic acids (in terms of lactic acid), 1.0 wt.%; ashes, 0.7 wt.%; 100 g of the product contains the following elements: sodium, 50 mg; potassium, 146 mg; calcium, 121 mg; magnesium, 14 mg; phosphorus, 90 mg; iron, 0.1 mg; vitamins, 1.59 mg. An energy value is 62.3 kcal per 100 g.
The product obtained may be used for oral administration in acute and severe forms of the diseases of the gastrointestinal tract.

### Example 14

The fermented-milk product Acidolact produced under the conditions identical to those described in Example 1, is separated under aseptic conditions into a liquid and a protein fraction. The liquid fraction is freeze-dried at the initial temperature of minus 50 ^{o}C and final temperature of minus 26 ^{o}C and a residual pressure of 13.3*10³ Pa for 24 hours.

The composition of the liquid fraction is as follows: water, 94.4 wt.%, dry substances, 5.6 wt.%, including: proteins, 0.35 wt.%; fats, 0.05 wt.%; carbohydrates, 1.5 wt.%; organic acids, etc., 3.2 wt.%, ashes, 0.5 wt.%. The dried Acidolact contains: water, 4 wt.%; dry substances, 96 wt.%, including: proteins, 6 wt.%; fats, 0.9 wt.%; carbohydrates, 27.0 wt.%; organic acids, 56.0 wt.%, ashes, 6.1 wt.%. 100 g of the product contains the following elements: sodium, 510 mg; potassium, 1400 mg; calcium, 1210 mg; magnesium, 130 mg; phosphorus, 800 mg; iron, 1.0 mg; vitamins, 15.3 mg. An energy value is 298 kcal/100 g.

The product obtained may be used as a dietotherapy and a treatment-and-prevention food, as well as a medicinal agent in surgery.

The protein fraction is used in dietary nourishment together with food additives.

### Example 15

An amount of 10 kg of a fat-free cow milk is sterilized at 120 ^{o}C for 15 minutes, cooled to 36 ^{o}C, then added thereto is 200 g of symbiotic microorganisms, consisting of 40.0 g of strains 317/392, 317/396 of the group Ep-2 Lactobacterium acidophilum taken in equal amounts, and 160 g of microorganisms of strain N.V. Ep 317/402 of Lactobacterium acidophilum. The symbiotic microorganisms are thoroughly mixed with the fat-free milk and allowed to stand for 5.5 hours. The obtained fermented product is cooled to +6 ^{o}C and allowed to stand again for 12 hours. An acidity of the liquid fermented milk is 85 ^{o}T.

The composition of the finished product as to the main ingredients is as follows: water, 91.9 wt.%; dry substances, 8.1 wt.%, including: proteins, 2.8 wt.%; fats, 0.1 wt.%; carbohydrates, 3.7 wt.%; organic acids, 0.8 wt.%; ashes, 0.7 wt.%. 100 g of the product contains the following elements: sodium, 51 mg; potassium, 144 mg; calcium, 118 mg; magnesium, 15 mg; phosphorus, 90 mg; iron, 0.1 mg. 1.08 mg of vitamins is contained per 100 g of the product. An energy value is 30 kcal per 100 g. One gram of the product contains 5*10⁸ microorganisms.

The product is used as a ferment for producing child's milk products and as drugs for rectal administration.

### Example 16

An amount of 1000 kg of milk with the fat content of 2.2 wt.% containing 3.0 wt.% of proteins, is sterilized at 136 ^{o}C for 5 seconds, cooled to 38 ^{o}C, then added thereto is 15 kg of symbiotic microoorganisms consisting of 5 kg of microorganisms of strain 317/395 of the group Ep-2 Lactobacterium acidophilum, and 10 kg of microorganisms of strain N.V. Ep 317/402 of Lactobacterium acidophilum. The symbiotic microorganisms are thoroughly mixed with the milk and allowed to stand at said temperature for 4 hours. The obtained fermented product is cooled to +6 ^{o}C and allowed to stand again for 18 hours. The product with an acidity of 120 ^{o}T is separated under aseptic conditions into a liquid and a protein fractions.

The composition of the liquid fraction is as follows: water, 93.8 wt.%; dry substances, 6.2 wt.%, including: proteins, 0.8 wt.%; fats, 0.2 wt.%; carbohydrates, 3.7 wt.%; organic acids, 1.0 wt.%; ashes, 0.5 wt.%; vitamins, 1.08 mg per 100 g of the product; 100 g of the product contains the following elements: sodium, 41 mg; potassium, 125 mg; calcium, 60 mg; magnesium, 6 mg; phosphorus, 80 mg; iron, 0.1 mg. An energy value is 23 kcal/100 g. One gram of the product contains 6*10⁶ microorganisms.

The composition of the protein fraction is as follows: water, 83.4 wt%; dry substances, 16.6 wt.%, including: proteins, 6.6 wt.%; fats, 6.0 wt.%; carbohydrates, 2.1 wt.%; organic acids, 1.0 wt.% , ashes, 0.9 wt.%; vitamines, 1.43 mg per 100 g. An energy value is 92 kcal/100 g. One gram of the product contains 6*10⁸ microorganisms. The liquid fraction is used for feeding and treating premature infants.

The protein fraction doped with carbohydrates is used as a complete food for infants over 6 months and patients of all age brackets.

### Example 17

An amount of 10 kg of a fat-free cow milk is heat treated at 120 ^{o}C for 20 minutes, cooled to 39 ^{o}C, then 320 g of symbiotic microorganisms consisting of 80.0 g of strain 317/396 of the group Ep-2 Lactobacterium acidophilum and 240 g of microorganisms of strain N.V. Ep 317/402 of Lactobacterium acidophilum are introduced into a cooled milk. Symbiotic microorganisms are thoroughly mixed and allowed to stand for 46 hours. The obtained fermented product is cooled to +8 ^{o}C and allowed to stand again for 18 hours. An acidity of a liquid fermented milk is 270 ^{o}T.

The product obtained is separated into a liquid and a protein fraction.

The composition of the finished liquid fraction is as follows: water, 94.4 wt.%; dry substances, 5.6 wt.%, including: proteins, 0.45 wt.%; fats, 0.05 wt.%; carbohydrates, 1.6 wt.%; organic acids, 3.0 wt.%; ashes, 0.5 wt.%. Vitamins - 1.54 mg/100 g. 100 gram of the product contains the following elements; sodium, 40 mg; potassium, 120 mg; calcium, 58 mg; magnesium, 14 mg; phosphorus, 75 mg; iron, 0.1 mg. An energy value is 20 kcal per 100 g. The composition of the protein fraction includes: water, 84.3 wt.%; dry substances, 15.9 wt.%, including: proteins, 11.0 wt.%; fats, 0.2 wt.%; carbohydrates, 1.6 wt.%; organic acids, 2.0 wt.%; ashes, 0.9 wt.%.

The product is used together with food additives as a dietotherapy.

### Example 18

The liquid fraction of the product produced as in Example 17 is freeze-dried at an initial temperature of minus 50 ^{o}C and a final temperature of minus 26 ^{o}C and at a residual pressure of 13.3*10³ Pa for 23 hours.

The obtained product retains its chemical and curative properties unaffected within two years.

The product is used as a functional dietary feeding.

The main chemical composition of the product is as follows: water, 4.0 wt.%; dry substances, 96 wt.%, including: proteins, 7.8 wt.%; fats, 0.5 wt.%; carbohydrates, 32.2 wt.%; organic acids, 50.5 wt.%; ashes, 5.0 wt.%. Vitamins, 15.4 mg/100 g; 100 g of the product contains the following elements: sodium, 510 mg; potassium, 1210 mg; calcium, 915 mg; magnesium, 133 mg; phosphorus, 810 mg; iron, 1.1 mg. An energy value is 346.4 kcal per 100 g.

### Example 19

The liquid fraction of the product produced as in Example 16 is freeze-dried at an initial temperature of minus 50 ^{o}C and a final temperature of minus 25 ^{o}C and at a residual pressure of 13.3*10³ Pa for 24 hours.

The obtained product retains its chemical and curative properties unaffected within two years.

The product is used as a functional and dietary feeding for children of all age brackets.

The main chemical composition of the product is as follows:

| | |
|---|---|
| water | 4.0 wt. % |
| dry substances | 96.0 wt. % |
| including: | |
| proteins | 12.3 wt. % |
| fats | 3.3 wt. % |
| carbohydrates | 58.8 wt. % |
| organic acids | 15.4 wt. % |
| ashes | 5.6 wt. % |
| vitamins | 10.86 mg/100 g. |

100 g of the product contains the following elements, mg: sodium, 390; potassium, 1275; calcium, 610; magnesium, 51; phosphorus, 802; iron, 1.1. An energy value is 362 kcal per 100 g. One gram of the product contains 3.0*10⁹ cells of microorganisms.

The product is used in a dietotherapy of premature infants.

### Example 20

The product produced in Example 3 is freeze-dried at an initial temperature of minus 50 ^{o}C and a final temperature of minus 26 ^{o}C and at a residual pressure of 13.3*10³ Pa for 24 hours.

The obtained product retains its chemical and curative properties unaffected within three months.

The product is used as a dry ferment for producing the product or for its oral administration.

The main chemical composition of the product is as follows: water, 4.0 wt.%; dry substances, 96 wt.%, including: proteins, 34.0 wt.%; fats, 0.6 wt.%; carbohydrates, 47.7 wt.%; organic acids, 7.2 wt.%; mineral substances, 6.8 wt.%.

An energy value is 352.6 kcal per 100 g.

The content of the various vitamins is 9.5 mg/100 g.

One gram of the product contains 2*10⁸ microorganisms.

### Example 21

An amount of 1000 kg of a cow milk containing 6.0 wt.% of fats, 4.0 wt.% of proteins, 5.0 wt.% of carbohydrates is pasteurized at 98 ^{o}C for 5 minutes, cooled to 36 ^{o}C, then added thereto is 20 kg of symbiotic microorganisms consisting of 4 kg of microorganisms of strain 317/392 of the group Ep-2 Lactobacterium acidophilum and 16 kg of microorganisms of strain N.V. Ep 317/402 of Lactobacterium acidophilum. Symbiotic microorganisms are thoroughly mixed with the milk and allowed to stand at 36 ^{o}C for 5.5 hours. The obtained fermented product is cooled to +6 ^{o}C and allowed to stand again for 12 hours. An acidity of the finished product is 65 ^{o}T.

The composition of the finished product as to the main ingredients is as follows: water, 84.2 wt.%; dry substances, 15.8 wt.%, including: proteins, 4.0 wt.%; fats, 6 wt.%; carbohydrates, 4.1 wt.%; organic acids, etc., 0.9 wt.%, ashes, 0.8 wt.%.

100 g of the product contains the following elements: sodium, 41 mg; potassium, 120 mg; calcium, 130 mg; magnesium, 14 mg; phosphorus, 80 mg; iron, 0.1 mg. An energy value is 90.0 kcal/100 g. 100 g of the product contains 2.54 mg of the varions vitamins and 4*10⁸ cells of microorganisms.

The product is used as a ferment for prophylactic and dietary feeding of patients of any age bracket beginning with 6 months of life.

### Example 22

The product produced in Example 21 is freeze-dried at an initial temperature of minus 50 ^{o}C and a final temperature of minus 26 ^{o}C and at a residual pressure of 13.3*10³ Pa for 23 hours.

The obtained product retains its chemico-biological and curative properties unaffected within two years.

The product is used as a treatment-and-prevention feeding.

The main chemical composition of the product is as follows: water, 4.0 wt.%; dry substances, 96.0 wt.%, including: proteins, 24.3 wt.%; fats, 36 wt.%; carbohydrates, 24.4 wt.%; organic acids, etc., 5.5 wt.%; ashes, 5.8 wt.%.

An energy value is 540 kcal per 100 g.

The content of the various vitamins is 10.3 mg/100 g.

Mineral substances: sodium, 400 mg; potassium, 1200 mg; calcium, 1200 mg; magnesium, 120 mg; phosphorus, 800 mg; iron, 1.0 mg.

One gram of the product contains 2*10⁸ microorganisms.

### Industrial Applicability

The present invention can find application in:
- commercial production of a liquid and a dry breast milk substitute "Acidolact-Narineh";
- batch production of a treatment-and-prevention product "Acidolact-Narineh" for humans of all ages;
- commercial production of liquid and dry ferments for fermented-milk products "Acidolact-Narineh";
- batch production of a treatment-and-prevention liquid and dry product "Acidolact-Narineh" for premature infants;
- commercial production of a liquid and a dry product "Acidolact-Narineh" used in medicine (surgery, gynecology, pediatrics) and for treating patients with acute and severe forms of the diseases of the gastrointestinal tract; improving an immune system of the organism;
- in veterinary-medicine practice.

## Claims

1. A fermented-milk product "Acidolact-Narineh", comprising proteins, fats, carbohydrates, organic acids, vitamins, water, and lactic-acid fermentation microorganisms of Lactobacterium acidophilum species, **characterized** in that it contains symbiotic microorganisms of at least one of the group Ep-2 strains with strain N.V. Ep 317/402, taken in a 1:1-4 ratio, respectively, in an amount of 10⁶ - 10⁹ symbiotic microorganisms per gram of the product, as lactic acid fermentation microorganisms of Lactobacterium acidophilum species, , wherein said ingredients are contained in the following amount:
| | |
|---|---|
| proteins | 0.35 - 34.0 wt. % |
| fats | 0.05 - 36.0 wt. % |
| carbohydrates | 1.5 - 70.0 wt. % |
| organic acids (in terms of lactic acid) | 0.6 - 56.0 wt. % |
| vitamins per 100 g | 0.95 - 15.9 wt. % |
| water | 3.7 - 94.6 wt. % |
| at an acidity of 60 - 280 ^{o}T. | |

2. A fermented-milk product "Acidolact-Narineh" according to Claim 1, **characterized** in that it further comprises microorganisms of Lactobacillus salivarius species.

3. A fermented-milk product "Acidolact-Narineh" according to Claim 1, **characterized** in that it contains proteins in an mount of 1.8 - 4.0 wt.%, fats, 0.05 - 6.0 wt.%, carbohydrates, 1.5 - 6.4 wt.%, organic acids (in terms of lactic acid), 0.6 - 3.2 wt.%, vitamins in an amount of 0.95 - 1.59 mg per 100 g, water, 84.2 - 91.90 wt.%.

4. A fermented-milk product "Acidolact-Narineh" according to Claim 1, **characterized** in that it contains proteins in an amount of 0.35 - 0.8 wt.%, fats, 0.05 - 0.20 wt.%,carbohydrates, 1.5 - 3.8 wt.%, organic acids (in terms of lactic acid), 0.7 - 3.15 wt.%, vitamins in an amount of 1.18 - 1.54 mg per 100 g, water, 93.20 - 94.6 wt.%.

5. A fermented-milk product "Acidolact-Narineh" according to Claims 3 and 4, **characterized** in that the proteins thereof contain the following essential amino acids, in percent of the sum total of the amino acids in the product:
| | |
|---|---|
| valine | 10.4 - 10.77 |
| isoleucine | 9.6 - 9.7 |
| leucine | 13.6 - 14.1 |
| lysine | 3.6 - 5.0 |
| methionine | 2.7 - 3.1 |
| threonine | 0.9 - 1.1 |
| tryptophan | 0.5 - 0.7 |
| phenylalanine | 8.4 - 8.5. |

6. A fermented-milk product "Acidolact-Narineh" according to Claims 3 and 4, **characterized** in that it contains the following vitamins per 100 g of a liquid product:
| | |
|---|---|
| Vitamin A | traces - 0.050 mg |
| Pantothenic acid | 0.10 - 0.190 mg |
| Vitamin C | 0.5 - 0.90 mg |
| Biotin | 0.002 mg |
| Vitamin E (tocopherol), traces | 0.2 mg |
| Nicotinic acid | 0.040 - 0.047 mg |
| Vitamin PP (niacin) | 0.09 - 0.14 mg |
| Vitamins of group B, | 0.218 - 0.315 mg |
| including: | |
| Vitamin B₁ (thiamine) | 0.04 - 0.07 mg |
| Vitamin B₂ (riboflavine) | 0.15 - 0.20 mg |
| Vitamin B₆ (pyridoxine) | 0.025 - 0.04 mg |
| Vitamin B_{c} (folic acid) | 0.003 - 0.005 mg. |

7. A fermented-milk product "Acidolact-Narineh" according to claim 3, **characterized** in that it contains:
| | |
|---|---|
| proteins | 24.0 - 34.0 wt. % |
| fats | 0.6 - 36.0 wt. % |
| carbohydrates | 26.8 - 50.0 wt. % |
| organic acids (in terms of lactic acid) | 4.4 - 36.4 wt. % |
| water | 3.7 - 4.0 wt. % |
| vitamins per 100 g of the product | 9.5 - 15.9 wt. %. |

8. A fermented-milk product "Acidolact-Narineh" according to Claim 4, **characterized** in that it contains:
| | |
|---|---|
| proteins | 6.0 - 12.9 wt. % |
| fats | 0.9 - 3.3 wt. % |
| carbohydrates | 26.7 - 70.2 wt. % |
| organic acids (in terms of lactic acid) | 15.4 - 56.0 wt. % |
| vitamins per 100 g of the product | 11.8 - 15.4 wt. % |
| water | 4.0 wt. %. |

9. A process for producing a fermented-milk product "Acidolact-Narineh" according to Claim 1, comprising the steps of:
heat-treating the milk at 95 - 140 ^{o}C; cooling it to a souring temperature;
introducing microorganisms of Lactobacterium acidophilum species; and
souring the milk to produce the end product,
**characterized** in that at least one known strain of group Ep-2 is used with strain N.V. Ep 317/402 as microorganisms of Lactobacterium acidophilum species , wherein strain Ep-2 is used in an amount providing species a 1:1-4 ratio to the amount of strain N.V. Ep 317/402, and souring is carried out to an acidity of 60 - 280 ^{o}T.

10. A process according to Claim 9, **characterized** in that it further comprises the step of:
introducing into a soured milk, microorganisms of Lactobacillus salivarius species taken in an amount providing a 1:1 ratio to the amount of microorganisms of Lactobacterium acidophilum species.

11. A process according to Claim 9, **characterized** in that it further comprises the step of:
separating a soured product into a liquid and a protein fraction under aseptic conditions.

12. A process according to Claims 9 and 11, **characterized** in that it further comprises the step of:
freeze-drying of a soured product up to a moisture content of not more than 4 wt.%.
